# EUROPÄISCHE PATENTANMELDUNG

(11) **EP 2 457 530 A1**
(43) Veröffentlichungstag der Anmeldung: **30.05.2012**
(21) Anmeldenummer: 11190511.3
(22) Anmeldetag: 24.11.2011
(51) Int. Cl.: A61B 17/80, A61B 17/86, A61C 13/00, A61L 27/28, C25D 11/02

(54) **Medizinisches Implantat**

(30) Priorität: 24.11.2010 DE 102010052231
(71) Anmelder: NORMED Medizin-Technik GmbH, 78532 Tuttingen (DE)
(72) Erfinder: Pech, Uwe, 78532 Tuttlingen (DE); Wörner, Roland, 78532 Tuttlingen (DE)
(74) Vertreter: Wagner, Kilian

(57) **Zusammenfassung**

Die Erfindung betrifft ein medizinisches, aus Titan oder einer Titanlegierung ausgebildetes Implantat (1), insbesondere Knochenplatte oder Schraube, mit einem einteiligen, eine farbige Oberfläche (4) aufweisenden Körper, wobei die Oberfläche (4) des Körpers einen eine erste Farbe aufweisenden flächigen ersten Farbbereich (2) und mindestens einen flächigen zweiten Farbbereich (3) aufweist, der eine von der ersten Farbe unterschiedliche zweite Farbe aufweist, erfindungsgemäß ist vorgesehen, dass der erste Farbbereich (2) eine die erste Farbe verursachende erste Oxidschicht (11) und der zweite Farbbereich (3) eine die zweite Farbe verursachende Oxidschicht (12) umfassen und dass sich die Dickenerstreckung der ersten Oxidschicht (11) des ersten Farbbereichs (2) von der Dickenerstreckung der zweiten Oxidschicht (12) des zweiten Farbbereichs (3) unterscheidet.

## Beschreibung

Die Erfindung betrifft ein medizinisches Implantat, insbesondere eine Knochenplatte, eine Schraube oder einen Kirschner-Draht, gemäß dem Oberbegriff des Anspruchs 1, sowie ein Sortiment mit mehreren Implantaten gemäß Anspruch 10. Ferner betrifft die Erfindung ein Verfahren zum Herstellen eines medizinischen Implantates gemäß Anspruch 11.

Aus der FR 1 080 370 sind Intramedullärimplantate bekannt, die unterschiedlich farbige Abschnitte aufweisen. Die Druckschrift schweigt sich jedoch hinsichtlich der konkreten Ausgestaltung der Farbschichten bzw. zu deren Auftragungsverfahren aus.

Die US 2005/0055097 A1 beschreibt implantierbare Platten als Teil eines Plattenpaketes, wobei es aus der Druckschrift bekannt ist, Plattenoberseite und Plattenunterseite unterschiedlich, beispielsweise rot und blau zu färben, um eine korrekte Orientierung der Platte zu gewährleisten. Auch diese Druckschrift schweigt hinsichtlich der Auftragungsweise der unterschiedlichen Farbschichten.

Aus der US 2010/0137921 A1 ist ein Fixierpin zum Fixieren einer Dentalschraubmatrize bekannt. In der Druckschrift ist beschrieben, dass es vorteilhaft ist, Farbkodierungen in Form von gefärbten Bändern vorzusehen, deren Beschaffenheit jedoch nicht näher erläutert ist. Üblicherweise handelt es sich um farbige Kunststoffringe, die in entsprechenden Aufnahmenuten fixiert werden können oder um aufgetragene Beschichtungen.

Die US 2008/0140127 A1 beschreibt eine Knochenplatte, bei welcher Schraubenlöcher unterschiedlich markiert sind, beispielsweise mittels einer Lasermarkierung oder einer Farbkodierung, wobei die Druckschrift nicht lehrt, wie die unterschiedlichen Farbmarkierungen aufgebracht werden.

Durch die US 2008/0097442 A1 ist ein Implantat zur Stabilisierung eines Knochens bekannt, wobei die Druckschrift lehrt, einen flexiblen Ring des mehrteiligen Implantates farbig auszugestalten.

Die DE 10 2007 005 417 A1 beschäftigt sich mit mehrteiligen Implantaten für die Anwendung an einer Wirbelsäule, wobei die einzelnen Plattenelemente des mehrteiligen Systems unterschiedlich farbig ausgestaltet sind, so dass der Anwender unmittelbar die Reihenfolge erfassen und umsetzen kann.

Das Vorsehen von zusätzlich farbigen Bauteilen, wie Farbringen ist problematisch. So können diese sich lösen und sind nur schwer sterilisierbar. Das Aufbringen von Lackierungen oder sonstigen Beschichtungen ist ebenfalls problematisch, da Wechselwirkungen der Farbe mit dem menschlichen Körper nicht auszuschließen sind. Darüber hinaus besteht die Gefahr des Ablösens.

Bekannt sind einfarbige medizinische Implantate aus Titanlegierungen. Die bekannten Implantate sind durch elektrolytische Bearbeitung mit einer einfarbigen Oxidschicht versehen. Die Oxidschicht überzieht dabei auch fakultativ vorgesehene Gravuren, mit dem beispielsweise der Implantattyp gekennzeichnet ist. Die Oxidbeschichtung dient zum Schutz des Implantates gegen Abnutzung. Ferner stellt die Oxidschicht einen, wenn auch begrenzten Schutz gegen galvanische Korrosion dar, für den Fall, dass das medizinische Implantat, beispielsweise eine Knochenplatte mit einem weiteren Implantat, aus einem weniger edlen Metall, beispielsweise einer Knochenschraube in Kontakt steht.

Bei den bisher in der Praxis zum Einsatz kommenden Implantaten kann es vorkommen, dass insbesondere im Hinblick auf bestimmte Operationsverfahren noch unerfahrene Operateure Schwierigkeiten mit der korrekten Anwendung, insbesondere Platzierung, Fixation etc. des Implantates haben.

Dies kann im schlimmsten Fall negative gesundheitsrelevante Nachteile für den Patienten nach sich ziehen.

Ausgehend von dem vorgenannten Stand der Technik liegt daher die Aufgabe zugrunde, verbesserte, einen mehrfarbigen sowie einstückigen Körper aufweisende Implantate anzugeben, durch deren Verwendung die Gefahr von Anwendungsfehlern minimiert wird. Insbesondere soll die Farbgebung beständig sein, d.h. es soll keine Gefahr eines Ablösens geben; darüber hinaus soll die Farbe möglichst biologisch und/oder chemisch inert sein. Ferner besteht die Aufgabe darin, ein Verfahren zur Herstellung entsprechend verbesserter Implantate anzugeben.

Diese Aufgabe wird hinsichtlich eines verbesserten Implantates mit den Merkmalen des Anspruchs 1 und hinsichtlich des Herstellungsverfahrens mit den Merkmalen des Anspruchs 17 gelöst.

Vorteilhafte Weiterbildungen der Erfindungen sind in den Unteransprüchen angegeben. In den Rahmen der Erfindung fallen sämtliche Kombinationen aus zumindest zwei von der in der Beschreibung, den Ansprüchen und/oder den Figuren offenbarten Merkmalen. Zur Vermeidung von Wiederholungen sollen vorrichtungsgemäß offenbarte Merkmale auch als verfahrensgemäß offenbart gelten und beanspruchbar sein. Ebenso sollen verfahrensgemäße offenbarte Merkmale als vorrichtungsgemäß offenbart gelten und beanspruchbar sein.

Der Erfindung liegt der Gedanke zugrunde, den einteiligen Implantatkörper nicht wie bisher einfarbig auszubilden, sondern mindestens zwei, vorzugsweise scharf begrenzte, Farbflächenbereiche vorzusehen, die eine voneinander unterschiedliche (mit dem menschlichen Auge unterscheidbare) Farbe aufweisen. Die Farbflächenbereiche können, zumindest näherungsweise, gleich groß bemessen sein oder einen voneinander unterschiedlichen Flächeninhalt aufweisen. Bevorzugt ist es, wenn die Flächenerstreckung jedes der mindestens zwei Farbbereiche mindestens 0,5 cm², vorzugsweise mindestens 1 cm^{2,} bevorzugt mindestens 2 cm², noch weiter bevorzugt mindestens 3 cm² beträgt. Dabei bezieht sich vorgenannte Flächenangabe bevorzugt auf einen zusammenhängenden (einteiligen) Flächenbereich, alternativ auf die Summe mehrerer einzelner Flächenbereiche der gleichen Farbe.

Erfindungsgemäß unterscheiden sich die Farbbereiche durch eine unterschiedliche Dickenerstreckung der jeweils für die Farbgebung verantwortlichen Schicht, insbesondere jeweils einer Oxidationsschicht. Bevorzugt beruht die unterschiedliche Farbgebung auf unterschiedlichen Interferenzeffekten der unterschiedlich dicken (insbesondere äußeren) Farbbereichsschichten, insbesondere Oxidationsschichten.

Die unterschiedlichen Farbbereiche können beispielsweise auf einer gemeinsamen Seite des Implantates, insbesondere der Knochenplatte angeordnet sein und/oder auf unterschiedlichen, insbesondere voneinander abgewandten Seiten. So ist es beispielsweise möglich, dass die Oberseite eine Knochenplatte gelb und die Unterseite einer Knochenplatte blau eingefärbt ist. Auch ist es möglich, auf der Oberseite und/oder der Unterseite einen gelben und einen blauen Bereich vorzusehen. Auch ist es denkbar, dass eine von zwei Seiten, beispielsweise die Oberseite einfarbig und die davon abgewandte Seite, in diesem Fall die Unterseite zweifarbig ausgebildet ist. Bevorzugt weisen die Farbbereiche eine scharfe (deutliche) Umfangskontur auf.

Durch die mehrfarbige Oberfläche können neben interessanten ästhetischen Effekten unterschiedliche Kennzeichnungsfunktionen realisiert werden, die helfen, Fehler bei der Anwendung des Implantates zu vermeiden.

Die unterschiedlichen Farbbereiche werden erfindungsgemäß durch unterschiedlich beschaffene Oxidationsschichten (Passivierungsschichten, Oberflächenschichten) des, vorzugsweise metallischen Implantatkörpers gebildet. Diese werden bevorzugt durch ein sogenanntes Farbanodisieren hergestellt. Die unterschiedliche Farbwirkung wird dabei bevorzugt, wie später noch erläutert werden wird, durch unterschiedliche elektrische Spannungen bei einer elektrolytischen Bearbeitung und/oder durch unterschiedliche zusammengesetzte Elektrolysebäder erreicht. Grundsätzlich ist das Farbanodisieren von Metallen in den internationalen für Luft- und Raumfahrt (Spezifikation LN 29747) beschrieben.

Bei der Farbwahl ist darauf zu achten, dass die mindestens zwei unterschiedlichen Farben deutlich mit dem menschlichen Auge unterscheidbar sind. Bevorzugt sind die zur Anwendung kommenden Farben in unterschiedliche tausender-Zahlenbereichen der RAL-Farbklassifikation angeordnet. Besonders bevorzugte Farbkombinationen sind gelb-blau, gelb-rot, gelb-grün, blau-rot, blaugrün, blau-gelb, rot-grün.

Wie bereits eingangs erwähnt, ist es besonders zweckmäßig, wenn die Flächenerstreckung des ersten und des zweiten Bereichs jeweils mindestens 0,5 cm², vorzugsweise mindestens 1 cm², bevorzugt mindestens 2 cm², noch weiter bevorzugt mindestens 3 cm² oder mehr betragen. Ganz besonders bevorzugt ist es, wenn jeweils ein zusammenhängender Flächenbereich vorerwähnte Flächenstreckungen aufweist.

Wie eingangs erwähnt, ist es erfindungsgemäß vorgesehen, dass die unterschiedliche Farbgebung der Farbbereiche durch unterschiedliche elektrolytisch, insbesondere durch anodische Oxidation, hergestellte Oxidationsschichten (A-nodisierungsschichten) bedingt ist. Bevorzugt ist die Dickenerstreckung jeder Oxidationsschicht aus einem Wertebereich zwischen etwa 20nm bis etwa 1500nm, bevorzugt zwischen 50nm, und 500nm, vorzugsweise zwischen 100nm und 400nm, besonders bevorzugt zwischen 150nm und 300nm gewählt. Unterschiedlich dicke und/oder zusammengesetzte bzw. aufgebaute Oxidationsschichten können durch die Wahl unterschiedlicher elektrischer Elektrolyse-spannungen, vorzugsweise aus einem Spannungsbereich zwischen 30V und 102V und/oder unterschiedlich zusammengesetzter Elektrolysebäder und/oder einer unterschiedlichen Behandlungsdauer gewählt werden.

Insbesondere unterscheiden sich die unterschiedlichen Oxidationsschichten durch ihre Dickenerstreckung, um einen unterschiedlichen Farbeindruck im menschlichen Auge hervorzurufen.

So ist es beispielsweise möglich in, insbesondere starken alkalischen Lösungen oder alternativ, in insbesondere schwach sauren Elektrolysebädern Oxidationsschichten herzustellen, wobei auch die Laugen oder Säuren unterschiedlich zusammengesetzt sein können.

Unterschiedlich dicke Oxidationsschichten können dadurch erzeugt werden, dass zueinander benachbarte und/oder zueinander beabstandete Oberflächenbereiche bei unterschiedlichen Spannungen und/oder in unterschiedlichen E-lektrolysebädern und/oder über eine unterschiedliche Zeitdauer elektrolytisch bearbeitet werden, so dass zwei unterschiedlich dicke und unterschiedlich farbige Oxidschichten auf der gemeinsamen Implantatkörperoberfläche entstehen. Alternativ ist es denkbar, dass sich die beiden Oberflächenbereiche überlappen, insbesondere dergestalt, dass ein zweiter Oberflächenbereich vollständig oberhalb des ersten, dann größeren Oberflächenbereichs angeordnet ist. Zunächst wird dann der erste Oberflächenbereich elektrolytisch bearbeitet, woraufhin nur ein Teilabschnitt dieses ersten Oberflächenbereichs mit identischen oder unterschiedlichen Parametern (Zeit, Spannung, Elektrolysebad) elektrolytisch bearbeitet wird, wobei dieser Teilabschnitt vollständig oder abschnittsweise den zweiten Oberflächenbereich bildet. Dadurch, dass ein Teilabschnitt des ersten Oberflächenbereichs mehrfach elektrolytisch bearbeitet wird, resultiert dort eine dickere Oxidschicht einer zweiten Farbe, die von der ersten Farbe, die der erste Oberflächenbereich in dem nicht nochmals elektrolytisch bearbeiteten Teilabschnitt aufweist, unterschiedlich ist.

Selbstverständlich ist es auch denkbar und sogar bevorzugt, unterschiedlich farbige Farbbereiche mit ein und derselben Elektrolysebadzusammensetzung herzustellen, also ggf. ausschließlich durch die Variation der Elektrolysespannung, ggf. gepaart mit der Variation weiterer Parameter, wie der Temperatur und/oder der Behandlungsdauer (Elektrolysedauer bzw. Spannungsbeaufschlagungsdauer).

Das erfindungsgemäße Vorsehen unterschiedlich dicker Oxidschichten als unterschiedliche Farbbereiche hat den Vorteil einer guten Stabilisierbarkeit. Gleichzeitig sind die Farbbereiche (Oxidschichten) im Körper chemisch und biologisch inert.

Bevorzugt ist es, das Implantat beim Elektrolysevorgang als Anode zu schalten. Besonders bevorzugt ist es, wenn als Katode eine Titankatode oder eine Titanlegierungskatode eingesetzt wird.

Wie bereits angedeutet, ist es wesentlich, dass die eine unterschiedliche Farbgebung aufweisenden Farbbereiche durch eine unterschiedliche Dickenerstreckung der für die Farbgebung maßgeblich verantwortlichen Schicht, insbesondere einer Oxidschicht unterscheiden. Dabei ist es besonders bevorzugt, wenn der Dickenunterschied mindestens, vorzugsweise im, insbesondere arithmetischen, Mittel (Durchschnitt) mindestens 10nm, vorzugsweise mindestens 20nm, weiter bevorzugt mindestens 50nm, noch weiter bevorzugt mindestens 100nm, oder mehr beträgt. Ganz besonders bevorzugt handelt es sich bei den mindestens zwei unterschiedlichen, insbesondere unterschiedlich dicken Oxidschichten um Titanoxidschichten, die eine identische oder voneinander unterschiedliche stöchiometrische Form (TIO, TIO₂, TI₂O₃, TI₃O₅, etc.) aufweisen können und die entweder die gleiche oder eine voneinander unterschiedliche Struktur aufweisen (amorph oder kristallin) mit den Phasen Anatas, Rotil und Brookit. Allgemein sind dünnere Oxidschichten eher amorph, während in dickeren Schichten der kristalline Anteil in der Regel mit der Schichtdicke ansteigt.

Bevorzugt beträgt die Dickenerstreckung einer blau wahrgenommenen Oxidschicht etwa 150nm, einer gelben Schicht etwa 200nm und einer grünen Schicht etwa 300nm.

Die Farbgebung der unterschiedlich dicken Oxidschichten basiert auf Interferenzeffekten. Mit zunehmender Schichtdicke bei Oxidschichten aus Titan werden eine Reihe von Farben erster bis vierter Ordnung durchlaufen, wobei die Farbe von der Schichtdicke und dem Brechungsindex abhängig ist.

Im Gegensatz zu anderen Oxiden, wie beispielsweise AL₂O3 hat TIO₂ einen hohen Brechungsindex von etwa 2,5 bis 2,7. Dies führt zu einem nicht unerheblichen Anteil an Reflektion an der Grenzfläche Oxidschicht/Luft der mit dem Anteil des von der Grenzfläche Oxidschicht/Luft, der mit dem Anteil des von der Grenzfläche Oxid/metallreflektierten Lichtes interferiert.

Die Erfindung macht sich die Erkenntnis zu Nutze, dass die Oxidschicht von Titan beim Anodisieren in Abhängigkeit des Elektrolyten, dessen Temperatur und vor allem der angelegten elektrischen Spannung eine charakteristische Dicke und damit auch Farbe annimmt.

Somit kann, beispielsweise bei konstanter Temperatur durch die Variation der elektrischen Spannung die Farbgebung verändert werden, wobei zwischen Spannung und Schichtdicke eine zumindest näherungsweise lineare Beziehung besteht.

Zusammengefasst ist es also gemäß der Erfindung vorgesehen, die unterschiedliche Farbgebung durch eine unterschiedliche Dickenerstreckung der für die Farbgebung verantwortlichen Schichten, insbesondere Titanoxidschichten bereitzustellen, wobei der Körper, um optimale Färbungen erreichen zu können, aus Titan oder einer Titanlegierung gefertigt ist.

Ganz besonders bevorzugt ist es, wenn der erste und der zweite Farbbereich unterschiedliche Funktionsbereiche und/oder Implantateigenschaften kennzeichnend ausbildet. So ist es beispielsweise denkbar, dass für unterschiedliche Implantate einer Implantatserie unterschiedlich mehrfarbig eingefärbt sind, um unterschiedliche Implantateigenschaften und/oder Verwendungszwecke zu kennzeichnen. Hierzu können die Flächenerstreckungen der Farbbereiche der verschiedenen Farbimplantate unterschiedlich gewählt und/oder die Farbkombinationen unterschiedlich sein. Besonders zweckmäßig ist es, wenn unterschiedliche Funktionsbereiche durch unterschiedliche Farben gekennzeichnet sind.

So kann beispielsweise ein erster flächiger Anlagebereich in einer ersten Farbe und ein flächiger Verschraubungsbereich (Fixationsbereich) in einer von der ersten Farbe (deutlich) unterschiedlichen zweiten Farbe gekennzeichnet sein. Zusätzlich oder alternativ können sich die Farbbereiche durch eine unterschiedliche Konturierung (Geometrie, Flächenform) unterscheiden. Ganz besonders zweckmäßig ist eine Ausführungsvariante, bei der der erste und der zweite Farbbereich unterschiedliche Anlagebereiche, zur Anlage an unterschiedlichen Knochenbereichen oder an unterschiedlichen Knochen kennzeichnend ausgebildet sind, wobei es besonders zweckmäßig ist, wenn die Kontur der Farbbereiche den Knochenbereichskonturen bzw. Knochenkonturen entspricht. Durch die Kennzeichnung unterschiedlicher Anlagebereiche wird eine korrekte Positionierung des vorzugsweise als Knochenplatte ausgebildeten Implantates sichergestellt.

Ebenso können Anwendungsfehler dadurch vermieden werden, dass der erste flächige Farbbereich einen ersten Intramedulärabschnitt des Implantates, also einen in einen Knochen, vorzugsweise in das Knochenmark, einzubringenden Abschnitt des Implantates, insbesondere einer Knochenplatte, kennzeichnend ausgebildet ist oder alternativ einen ersten Extramedulärabschnitt, also einen außen an einem Knochen anlegbaren Bereich des Implantates.

Besonders zweckmäßig ist es, wenn der zweite Farbbereich einen zweiten, von dem ersten Intramedulärabschnitt unterschiedlichen Intramedulärabschnitt oder einen zweiten, von dem ersten Extramedulärabschnitt unterschiedlichen Extramedulärabschnitt kennzeichnend ausgebildet ist, so dass das Implantat selbst eine Anleitung für die korrekte Anwendung darstellt.

Eine weitere Anwendungsmöglichkeit ist die Kennzeichnung von Steril- und Unsterilbereiche des Implantates, insbesondere der Knochenplatte, bevorzugt derart, dass der erste flächige Farbbereich durch seine erste Farbe einen ersten Sterilabschnitt und/oder zweite flächige Farbbereich einen Unsterilabschnitt kennzeichnet ausgebildet ist.

Gemäß einer besonders bevorzugten Ausführungsvariante kennzeichnen die mindestens zwei unterschiedlichen flächigen Farbbereiche unterschiedliche Schraubenlöcher oder unterschiedliche Gruppen von Schraubenlöchern, beispielsweise derart, dass mindestens zwei Schraubenlöcher, zumindest teilweise, von unterschiedlichen Farben umringt sind. Durch die Farbkennzeichnung kann beispielsweise eine Fixationsreihenfolge angegeben werden, also eine Reihenfolge, in welcher die Schrauben in die entsprechend gekennzeichneten Schraubenlöcher eingebracht werden sollen. Insbesondere durch die Kennzeichnung unterschiedlicher Schraubenlöchergruppen können unterschiedliche Verschraubungsvarianten, insbesondere für unterschiedliche Anwendungszwecke gekennzeichnet werden, wobei sich die unterschiedlichen Schraubengruppen durch mindestens ein Schraubenloch unterscheiden. Für den Fall, dass mindestens ein Schraubenloch zu mindestens zwei Schraubengruppen gehörig sein soll, sollte dieses Schraubenloch, beispielsweise durch das Vorsehen von zwei Halbringen oder zwei konzentrischen Ringen mit zwei unterschiedlichen flächigen Farbbereichen gekennzeichnet sein.

Eine weitere bevorzugte Möglichkeit zur Minimierung von Anwendungsfehlern besteht in der Kennzeichnung unterschiedlicher Skalierungen durch die unterschiedlichen flächigen Farbbereiche.

So kann ein erster, beispielsweise blauer Farbbereich eine erste bevorzugte Bohrtiefe und ein zweiter, beispielsweise gelber Farbbereich eine zweite, insbesondere von der ersten Bohrtiefe unterschiedliche Bohrtiefe kennzeichnen, um dem Operateur entsprechend bei dem Einbringen von Knochenbohrungen einzuleiten.

So kann beispielsweise ein erster, insbesondere gelber flächiger Farbbereich einen Metatarsale Medial-Anlagebereich und ein zweiter, beispielsweise blauer flächiger Farbbereich einen Metatarsale Proximal-Anlagebereich einer Knochenplatte kennzeichnen.

Bei einem alternativen Ausführungsbeispiel ist es denkbar, dass bei einer Knochenplatte ein erster, beispielsweise roter flächiger Farbbereich den Metatarsale-Anlagebereich und ein zweiter, beispielsweise grüner flächiger Farbbereich den Keilbein-Anlagebereich kennzeichnet. Auch sind Ausführungsvarianten mit drei oder mehr unterschiedlichen flächigen Farbkennzeichnungen (Farbbereichen) realisierbar. So kann beispielsweise ein erster, insbesondere blauer flächiger Farbbereich den Tibia-Anlagebereich, ein zweiter, beispielsweise gelber flächiger Farbbereich den Talus-Anlagebereich und ein dritter, beispielsweise grüner flächiger Farbbereich den Calcaneus-Anlagebereich kennzeichnen. Auch ist es möglich, durch einen ersten Farbbereich einen biegsamen Implantatbereich, beispielsweise den biegsamen Bereich einer Knochenplatte zu kennzeichnen und/oder mit dem zweiten, von dem ersten Farbbereich unterschiedlichen (unterschiedlich farbigen) flächigen Farbbereich einen starren Implantatbereich.

Ferner können Anwendungsfehler dadurch vermieden werden, dass die unterschiedlichen Farbbereiche, insbesondere bei gemeinsamer Berücksichtigung bzw. Betrachten eine vorgegebene Orientierung des Implantates, insbesondere der Knochenplatte kennzeichnen, so beispielsweise die Orientierung dorsal/plantar, insbesondere bei Fußknochenplatten, wobei beispielsweise die Dorsal zu orientierende Plattenseite gelb oder rot und die davon abgewandte, plantar zu orientierende Plattenseite blau eingefärbt ist.

Zudem kann die Orientierung proximal/distal oder insbesondere bei Handknochenplatten palmar/dorsal durch entsprechende unterschiedliche Farben (flächige Farbbereiche) gekennzeichnet sein.

Auch ist es möglich, einen Trennbereich eines Implantates, insbesondere einer Knochenplatte zu kennzeichnen.

Unter einem Trennbereich wird hierbei ein Bereich verstanden, in welchem das Implantat, insbesondere die Knochenplatte, insbesondere durch einen von außen wirksamen mechanischen Eingriff deformiert, insbesondere in zwei Teile unterteilt werden kann, wodurch die Funktionalität des Implantates veränderbar ist, um beispielsweise eine von einer Standard-OP-Variante abweichende OP-Variante durchführen zu können.

Die Erfindung betrifft auch ein Sortiment aus mindestens zwei unterschiedlichen Implantaten, wobei sich die beiden Implantate beispielsweise durch unterschiedliche Farbkombinationen auszeichnen, und/oder durch eine unterschiedliche Anzahl von unterschiedlichen Farben. Zusätzlich oder alternativ können die Farbbereiche bei den unterschiedlichen Implantaten unterschiedlich groß gewählt und/oder unterschiedlich konturiert sein.

Die Erfindung führt auch auf ein Verfahren zum Herstellen eines nach dem Konzept der Erfindung ausgebildeten medizinischen Implantates mit einem einteiligen Implantatkörper, der sich durch flächige, unterschiedlich farbige Farbbereiche auszeichnet.

Kern des Verfahrens ist es, den Implantatkörper an mindestens zwei Flächenbereichen (unterschiedlich) elektrolytisch zu bearbeiten, um so zwei Oxidschichtflächen zu erhalten, die sich durch eine unterschiedliche inhärente Farbgebung unterscheiden. Anders ausgedrückt ist es Kern des Verfahrens durch Variation von Bearbeitungsparametern einer elektrolytischen Bearbeitung der Oberfläche des Implantatkörperrohlings zweiflächige Farbbereiche zu schaffen, die sich jeweils durch eine farbige Oxidschicht auszeichnen, wobei sich die Farbgebung der flächigen Oxidschichten für das Auge erkennbar unterscheidet.

Zur Herstellung unterschiedlich gefärbter Flächen ist es möglich, zunächst einen ersten, vorzugsweise in seiner Gesamtheit, später den ersten Farbbereich der ersten Farbe ausbildenden Oberflächenbereich bei einer ersten elektrischen Spannung über eine erste Zeitdauer in einem ersten Elektrolysebad elektrolytisch zu bearbeiten. In einem weiteren Schritt wird dann ein zweiter, vorzugsweise in seiner Gesamtheit, später den zweiten Farbbereich der zweiten Farbe ausbildender Oberflächenbereich elektrolytisch bearbeitet, wobei sich in diesem Fall bevorzugt erster und zweiter Oberflächenbereich nicht überlappen.

In diesem Fall ist es zur Erzeugung unterschiedlich dicker und damit unterschiedlich farbiger Oxidschichten erforderlich, dass die elektrolytische Bearbeitung des zweiten Oberflächenbereichs bei einer von der ersten elektrischen Spannung unterschiedlichen zweiten elektrischen Spannung und/oder in einem von dem ersten Elektrolysebad unterschiedlichen Elektrolysebad und/oder über eine von der ersten Zeitdauer unterschiedliche Zeitdauer erfolgt.

Alternativ ist es möglich, dass der zweite Oberflächenbereich zum Teil oder vollständig oberhalb des ersten Oberflächenbereichs erzeugt wird. In diesem Fall wird also zunächst auch ein erster Oberflächenbereich elektrolytisch bearbeitet, wobei in diesem Fall der erste Oberflächenbereich vorzugsweise, jedoch nicht zwingend, von der Gesamtoberfläche des Implantatkörperrohlings gebildet wird. Die elektrolytische Bearbeitung kann bei einer ersten elektrischen Spannung und über eine erste Zeitdauer und in einem ersten Elektrolysebad erfolgen, wobei als Resultat der gesamte erste Oberflächenbereich die erste Farbe aufweist. Nun erfolgt die Bearbeitung des den ersten Oberflächenbereich teilweise überdeckenden zweiten Oberflächenbereichs, wobei folglich in dem Überlappungsbereich die zweite Farbe erzeugt wird, da die Oxidschicht in dem zweiten Oberflächenbereich durch die zusätzliche elektrolytische Bearbeitung dicker wird als in dem von der weiteren Oberflächenbehandlung ausgesparten Teilabschnitt des ersten Oberflächenbereichs. Nur dieser ausgesparte Oberflächenbereich bildet den ersten Farbbereich der ersten Farbe, wobei hingegen der Teilabschnitt des ersten Oberflächenbereichs der Bestandteil des zweiten Oberflächenbereichs ist durch die zusätzliche bzw. weitere elektrolytische Bearbeitung eine dickere Oxidschicht und damit eine zweite Farbe erhält. Dieser mindestens zweifach elektrolytisch bearbeitete Teilabschnitt des ersten Oberflächenbereichs bildet dann vorzugsweise den zweiten Farbbereich der zweiten Farbe. Bei sich überlappenden Oberflächenbereichen ist es nicht notwendig, dass die zweite elektrische Spannung von der ersten elektrischen Spannung abweicht und/oder dass zweite Elektrolysebad nicht dem ersten Elektrolysebad entspricht und/oder dass die zweite Zeitdauer unterschiedlich ist von der ersten Zeitdauer.

Wesentlich ist lediglich, dass ein Teilbereich des ersten Oberflächenbereichs der den zweiten Oberflächenbereich bildet oder zumindest zu diesem gehört mindestens einer zusätzlichen elektrolytischen Bearbeitung unterzogen wird mit dem Ziel, eine dickere Oxidschicht der zweiten Farbe zu erzeugen. Der zweite Oberflächenbereich kann vollständig oberhalb des vorzugsweise eine größere Flächenerstreckung aufweisenden ersten Oberflächenbereichs angeordnet sein, oder seitlich über diesen herausragen, wobei in dem herausragenden Teilabschnitt von dem überlappenden Teilabschnitt unterschiedliche Farbeffekte erzielbar sind.

Damit der Teilabschnitt des ersten Oberflächenbereichs, der nicht einer weiteren elektrolytischen Bearbeitung unterzogen werden soll nicht in Kontakt kommt mit dem Elektrolysebad kann entweder der Implantatkörperrohling nur mit dem nochmals zu bearbeitenden Teilabschnitt des ersten Oberflächenbereichs, also mit dem zweiten Oberflächenbereich eingetaucht werden und der nicht zu bearbeitende Teilabschnitt bleibt oberhalb des Elektrolysebades und/oder der zu schützende bzw. nicht zu bearbeitende Abschnitt des ersten Oberflächenbereichs wird geeignet geschützt, beispielsweise durch eine im Nachgang wieder zu entfernende Schutzschicht, z.B. einen Schutzanstrich und/oder durch ein Klebeband.

Ganz besonders zweckmäßig ist es, wenn der Implantatkörper vor der elektrolytischen Bearbeitung von einer natürlichen Oxidschicht durch eine entsprechende chemische Behandlung befreit wird, um dann in einem weiteren Schritt definiert mindestens zwei unterschiedlich farbige Oxidschichten aufbringen zu können, die sich, wie später noch erläutert werden wird, bevorzugt durch ihre für die Farbgebung verantwortlichen Dickenerstreckung unterscheiden.

Besonders bevorzugt ist es, wenn während der elektrolytischen Bearbeitung das Implantat als Anode geschaltet ist. Besonders zweckmäßig wird als Kathode eine Titanelektrode oder eine Titanlegierungselektrode eingesetzt, um die Oxidschicht zu erzeugen, wobei unterschiedlich farbige Oxidschichten auch mit ein und derselben Kathode herstellbar sind, wobei allerdings der Aufbau der Oxidschichten durch unterschiedliche Kathodenmaterialien beeinflusst werden kann.

Besonders bevorzugt ist es, wenn die mindestens zwei unterschiedlichen Oxidschichtflächen und damit die unterschiedlichen flächigen Farbbereiche bei unterschiedlichen elektrischen (Elektrolyse-Spannungen) durchgeführt werden, und zwar mit demselben, einem gleichen oder einem unterschiedlich zusammengesetzten Elektrolysebad.

Bevorzugt unterscheiden sich die Elektrolysespannungen, insbesondere bei gleicher Behandlungsdauer (Elektrolysedauer), zum Erhalten unterschiedlich farbiger Farbbereiche um mindestens 1V, vorzugsweise um mindestens 2V, noch weiter bevorzugt um mindestens 3, 4, 5, 6, 7, 8, 9, 10 oder mehr Volt. Ziel ist es, durch die unterschiedlichen Elektrolysespannungen insbesondere bei gleicher Elektrolysedauer, unterschiedlich dicke, die unterschiedlichen flächigen Farbbereiche bildenden Oxidschichten, insbesondere Titanoxidschichten, zu erhalten, wobei die unterschiedliche Farbgebung bevorzugt auf Interferenzeffekten beruht und die Farbgebung von der Dicke der Oxidschicht abhängig ist. Dabei können die Oxidschichten grundsätzlich gleich oder ähnlich aufgebaut sein oder sich in ihrem Aufbau, insbesondere in ihrer Stöchiometrie und/oder Struktur unterscheiden.

Zusätzlich oder alternativ ist es denkbar zum Erhalt unterschiedlich dicker Oxidschichten, die Elektrolyse in den unterschiedlichen Bereichen unterschiedlich lange durchzuführen. Bevorzugt beträgt der Unterschied in der Behandlungsdauer mindestens 30s, vorzugsweise mindestens 1 min, 2min, 3min, 4min, 5min oder mehr.

Wesentlich ist jedenfalls, dass die Oxidschichten der unterschiedlichen Farbbereiche eine voneinander unterschiedliche Dickenerstreckung aufweisen. Das einfallende weiße Licht wird beim Eintritt in die jeweilige Oxidschicht gebrochen, in seine Spektralfarben zerlegt und am Übergang Oxidschicht/Metall reflektiert. Im Vergleich mit dem direkt an der Grenzfläche Luft/Oxidschicht reflektierten Lichts ergeben sich Wegunterschiede, die von der Wellenlänge und der Oxidschichtdicke abhängen. Ist der Gangunterschied in der Oxidschicht mit der Dicke gerade ein ganzes Vielfaches der eingestrahlten Wellenlänge Lambda, so resultiert hieraus konstruktive Interferenz, also eine Verstärkung. Im Reflektionsspektrum zeigt sich dies durch ein Maximum, während man im Gegensatz dazu bei einem Minimum destruktive Interferenz hat.

Zusätzlich oder alternativ können unterschiedliche Elektrolysebäder zur Erzeugung der unterschiedlich farbigen Oxidschichten (Farbbereiche) Verwendung finden.

Besonders zweckmäßig ist es, wenn bei den unterschiedlichen elektrolytischen Bearbeitungen sichergestellt ist, dass der jeweils nicht zu behandelnde Farbbereich nicht in das Elektrolysebad getaucht ist und/oder dass der nicht zu behandelnde Bereich mit einer Schutzschicht, insbesondere einem Schutzklebeband überzogen ist.

Anstelle (oder zusätzlich dazu) eines chemisch resistenten Schutzklebebandes können chemisch resistente, plastisch verformbare Massen umgesetzt werden oder eine Teilbeschichtung, die nach der elektrolytischen Behandlung eines Teilbereichs vor der elektrolytischen Behandlung des durch die Teilbeschichtung geschützten Bereichs wieder, vorzugsweise vollständig, entfernt wird.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: am Beispiel einer Knochenplatte ein Implantat mit einer Oberfläche, die in zwei gleiche Farbbereiche unterschiedlicher Farbgebung aufgeteilt ist,

- Fig. 2: eine alternative Knochenplatte, bei der zwei Gruppen von Schraubenlöchern die unterschiedlich farbigen flächigen Farbbereichen in Ringform gekennzeichnet sind,
- Fig. 3: eine Knochenplatte, bei der durch unterschiedlich farbige Farbbereiche eine Verschraubungsreihenfolge gekennzeichnet ist,
- Fig. 4: eine Knochenplatte, bei der zwei Gruppen von Schraubenlöchern unterschiedlich farbig gekennzeichnet sind, wobei ein Schraubenloch mit zwei Farbkennzeichnungen versehen ist, um aufzuzeigen, dass dieses Schraubenloch sowohl zur einen als auch zur anderen Gruppe von Schraubenlöchern gehört und
- Fig. 5: eine Knochenplatte mit drei unterschiedlich farbigen flächigen Farbbereichen und,
- Fig. 6: eine Schnittansicht eines Implantates mit zwei flächigen Farbbereichen, bei denen die für die unterschiedliche Farbgebung verantwortlichen Oxidschichten eine voneinander unterschiedliche durchschnittliche Dickenerstrechung aufweisen.

In den Figuren sind gleiche Elemente und Elemente mit der gleichen Funktion mit den gleichen Bezugszeichen gekennzeichnet.

An dieser Stelle sei angemerkt, dass in den Figuren lediglich Knochenplatten dargestellt sind. Hierauf ist die Erfindung jedoch nicht beschränkt. Die Knochenplatten stehen stellvertretend für einteilige, mehrfarbige Implantatkörper.

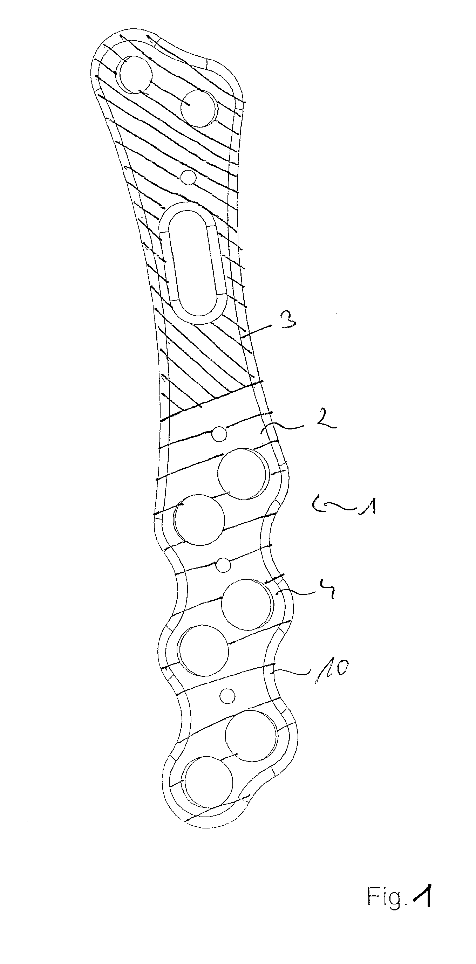

In Fig. 1 ist stellvertretend für ein medizinisches Implantat 1 eine Knochenplatte bestehend aus einem einteiligen Körper 10 gezeigt. Durch unterschiedliche Schraffierungen sind zwei unterschiedlich farbige, flächige Farbbereiche gekennzeichnet, nämlich ein erster, in der Zeichnungsebene unterer flächiger, z.B. blauer Farbbereich 2 und ein daran angrenzender, in der Zeichnungsebene oberer flächiger, zweiter, z.B. gelber Farbbereich 3. Die unterschiedliche Farbgebung der beiden Farbbereiche 2, 3 ist auf eine unterschiedliche elektrolytische Bearbeitung zurückzuführen - anders ausgedrückt handelt es sich bei den Farbbereichen um Oxidschichten mit einer voneinander unterschiedlichen inhärenten Farbgebung.

Die farbige Oberfläche 4 des Implantates 1 signalisiert auf die gezeigte Weise beispielsweise eine bevorzugte Implantatorientierung, hier dorsal/distal oder zwei unterschiedliche Anlagenbereiche zur Anlage an unterschiedlichen Knochenbereichen oder an unterschiedlichen Knochen.

Die beiden Farbbereiche 2, 3 unterscheiden sich durch die Dickenerstreckung der für die jeweilige Farbgebung verantwortlichen Titanoxidschicht.

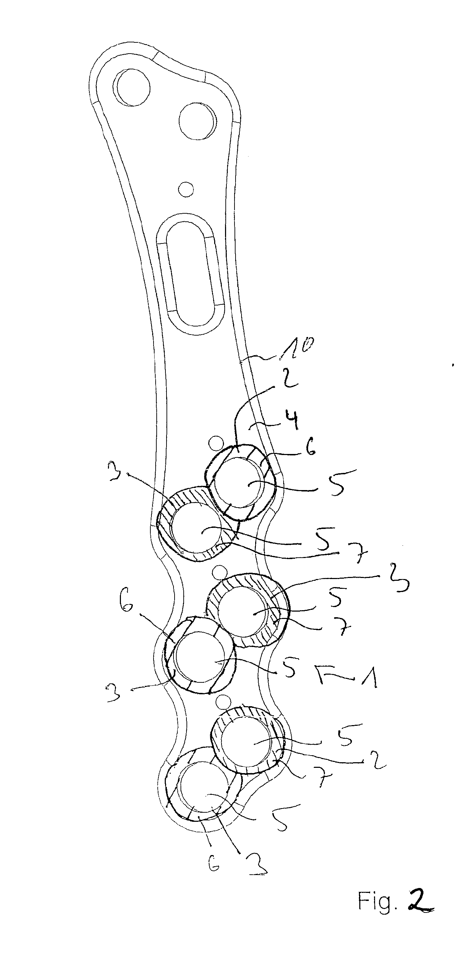

In Fig. 2 ist ein weiteres Implantat 1 dargestellt. Zu erkennen ist auch hier eine mehrfarbige Oberfläche 4. In dem Implantat 1 sind Schraubenlöcher 5 eingebracht, die in zwei Gruppen unterteilt sind, nämlich eine erste Gruppe 6 und eine zweite Gruppe 7 von Schraubenlöchern 5 mit jeweils drei Schraubenlöchern. Die beiden Gruppen 6, 7 sind durch unterschiedlich farbige flächige Farbbereiche 2, 3 gekennzeichnet, die durch unterschiedliche Schraffuren dargestellt sind. Jeder Farbbereich 2, 3 weist drei voneinander beabstandete, ringförmige Flächen auf, die die zur jeweiligen Gruppe 6, 7 gehörenden Schraubenlöcher 5 koaxial umgeben. Die Gruppen 6, 7 von Schraubenlöchern 5 können alternativ Anwendung finden.

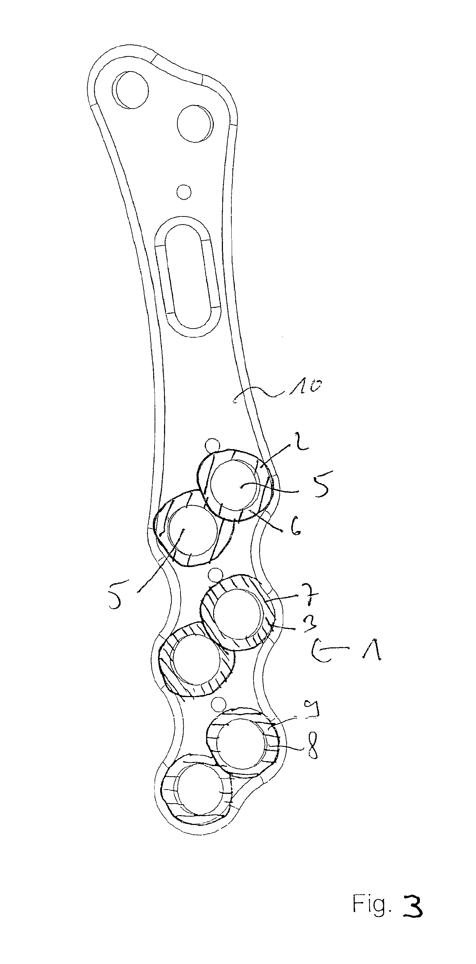

Bei dem Ausführungsbeispiel gemäß Fig. 3 ist ein alternatives Implantat 1 dargestellt, wobei jeweils zwei nebeneinander angeordnete Schraubenlöcher 5 zu einer gemeinsamen Gruppe von Schraubenlöchern gehören. Insgesamt sind drei solche Gruppen 6, 7, 8 vorgesehen, wobei jeder Gruppe ein flächiger Farbbereich 2, 3, 9 zugeordnet ist.

Auch bei dem Ausführungsbeispiel gemäß Fig. 2 weisen die Farbbereiche 2,3 eine unterschiedliche Dickenerstreckung auf - anders ausgedrückt sind die für die unterschiedlichen Farbgebungen der Farbbereiche 2,3 verantwortlichen Oxidschichten unterschiedlich dick, wobei die unterschiedliche Dicke bevorzugt durch unterschiedlich große Elektrolysespannungen und/oder unterschiedliche Elektrolysebehandlungszeiten erreicht werden.

Das in Fig. 3 gezeigte Implantat zeichnet sich also durch drei flächige Farbbereiche 2, 3, 9 aus, die sich in ihrer Farbgebung deutlich unterscheiden. Grundsätzlich ist es möglich, noch weitere Farbbereiche vorzusehen - so kann beispielsweise der Flächenbereich um die konzentrisch angeordneten Kreisringe in einer vierten Farbe gestaltet sein. Bevorzugt stellt die flächige Farbkennzeichnung eine Fixierreihenfolge dar, in welcher die drei Gruppen 6, 7, 8 von Schraubenlöcher 5 fixiert werden sollten.

Auch bei dem Ausführungsbeispiel gem. Fig. 3 unterscheiden sich die drei flächige Farbbereiche 2, 3, 9 durch die Dickenerstreckung der für die Farbgebung verantwortlichen, elektrolytisch erzeugten Oxidschichten. Das gleiche gilt auch für die Dickenerstreckung der unterschiedlich farbigen Farbbereiche in den noch folgenden Ausführungsbeispielen.

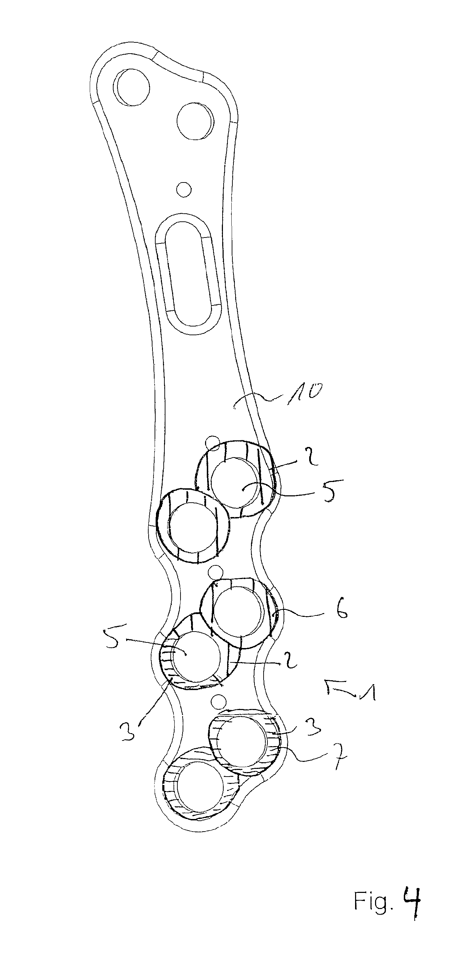

Bei dem in Fig. 4 gezeigten Implantat sind zwei Gruppen 6, 7 von Schraubenlöchern mit unterschiedlichen flächigen Farbbereichen 2, 3 gekennzeichnet, wobei das von in der Zeichnungsebene unten gezählte dritte Schraubenloch 5 mit zwei halbkreisringförmigen, unterschiedlichen Farbbereichen 2, 3 gekennzeichnet ist. Hiermit soll zum Ausdruck gebracht werden, dass das Schraubenloch 5 zu beiden Gruppen 6, 7 gehörig sein soll.

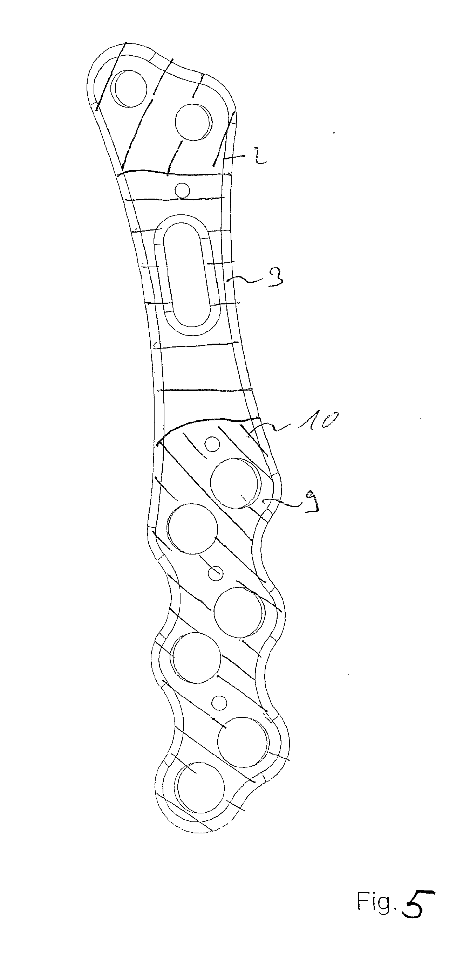

In Fig. 5 sind drei unterschiedliche Funktionsbereiche durch drei unterschiedlich farbige, flächige Farbbereiche 2, 3, 9 gekennzeichnet. Bei dem ersten in der Zeichnungsebene oberen flächigen Farbbereich 2 handelt es sich um einen ersten Fixierbereich mit zwei Schraubenlöchern, die für eine erste Art von Schrauben ausgebildet sind.

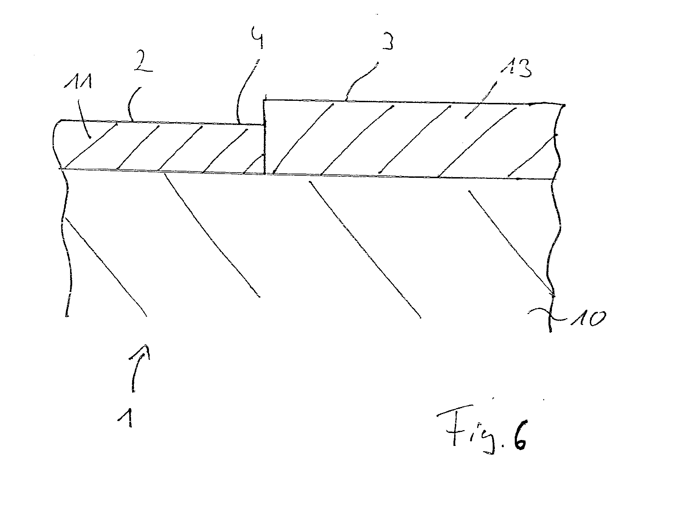

Bei dem zweiten farbigen Flächenbereich 3 handelt es sich um einen Langlochbereich und bei dem dritten flächigen Farbbereich 9 um einen weiteren Fixierbereich für eine alternative Art von Knochenschrauben, die von der Art, der mit dem ersten Fixierbereich verwendbaren Knochenschrauben unterschiedlich ist. In Fig. 6 ist in einer schematischen Schnittansicht ein Implantat 1 gezeigt, welches mit einer Oberfläche 4 versehen ist, die einen ersten flächigen Farbbereich 2 mit einer ersten Farbe und einen zweiten flächigen Farbbereich 3 mit einer zweiten, von der ersten Farbe deutlich unterschiedlichen Farbe aufweist. Die beiden unterschiedlich farbigen, flächigen Farbbereiche 2, 3 sind gebildet von jeweils einer Oxidschicht, nämlich einer ersten Oxidschicht 11 und einer zweiten Oxidschicht 12, die elektrolytisch auf den Körper 10 des Implantates 1 erzeugt wurden. Der Körper 10 besteht beispielsweise aus Titan oder einer Titanlegierung. Die unterschiedliche Farbgebung der Flächenbereiche 2, 3 wird erzielt durch eine unterschiedliche Dickenerstreckung der ersten und der zweiten Oxidschicht 11, 12. Bei den beiden Oxidschichten handelt es sich um Titanoxidschichten, die die gleiche Stöchiometrie und die gleiche Struktur oder eine voneinander unterschiedliche Stöchiometrie und/oder einer voneinander unterschiedliche Struktur aufweisen können. Verantwortlich für die unterschiedliche Dickenerstreckung der ersten und der zweiten Oxidschicht 11, 12 sind die unterschiedlichen bei ihrer Herstellung angelegten elektrischen Spannungen, und/oder unterschiedlich lange Behandlungszeiten.

In einem von dem Körper 10 abgelösten Zustand können die Oxidschichten 11, 12 beispielsweise transparent, also mehr oder weniger farblos sein. Die Farbgebung der Oxidschichten 11, 12 bzw. vielmehr der Farbbereiche 2, 3 beruht auf Interferenzerscheinungen, wobei für die Farbgebung bzw. die Farberscheinungen in erster Linie die jeweilige Dickenerstreckung der Oxidschichten 11, 12 verantwortlich ist, wobei sich die Dickenerstreckung hier, wie bereits erläutert, unterscheidet und zwar bevorzugt um mindestens 20nm, vorzugsweise mindestens 50nm, noch weiter bevorzugt um mindestens 100nm oder mehr.

### Bezugszeichenliste

- 1: Implantat
- 2: erster flächiger Farbbereich
- 3: zweiter Farbbereich
- 4: Oberfläche
- 5: Schraubenlöcher
- 6: erste Gruppe
- 7: zweite Gruppe
- 8: dritte Gruppe
- 9: dritter flächiger Farbbereich
- 10: Körper
- 11: erste Oxidschicht
- 12: zweite Oxidschicht

## Patentansprüche

1. Medizinisches, aus Titan oder einer Titanlegierung ausgebildetes Implantat (1), insbesondere Knochenplatte oder Schraube, mit einem einteiligen, eine farbige Oberfläche (4) aufweisenden Körper, wobei die Oberfläche (4) des Körpers einen eine erste Farbe aufweisenden flächigen ersten Farbbereich (2) und mindestens einen flächigen zweiten Farbbereich (3) aufweist, der eine von der ersten Farbe unterschiedliche zweite Farbe aufweist,
**dadurch gekennzeichnet,**
**dass** der erste Farbbereich (2) eine die erste Farbe verursachende erste Oxidschicht (11) und der zweite Farbbereich (3) eine die zweite Farbe verursachende Oxidschicht (12) umfassen, und dass sich die Dickenerstreckung der ersten Oxidschicht (11) des ersten Farbbereichs (2) von der Dickenerstreckung der zweiten Oxidschicht (12) des zweiten Farbbereichs (3) unterscheidet.

2. Implantat nach Anspruch 1,
**dadurch gekennzeichnet,**
**dass** der erste und der zweite Farbbereich (2, 3) Implantateigenschaften oder unterschiedliche Funktionsbereiche oder, kennzeichnend ausgebildet, insbesondere konturiert und/oder angeordnet, sind.

3. Implantat nach einem der Ansprüche 1 oder 2,
**dadurch gekennzeichnet,**
**dass** der erste und der zweite Farbbereich (2, 3) unterschiedliche Anlagebereiche zur Anlage an unterschiedlichen Knochenbereichen oder Knochen kennzeichnend ausgebildet sind.

4. Implantat nach einem der vorhergehenden Ansprüche,
**dadurch gekennzeichnet,**
**dass** der erste Farbbereich (2) einen ersten Intramedulärabschnitt oder einen ersten Extramedulärabschnitt des Körpers und/oder der zweite Farbbereich (3) einen zweiten Intramedulärabschnitt oder einen zweiten Extramedulärabschnitt des Körpers kennzeichnend ausgebildet sind/ist.

5. Implantat nach einem der vorhergehenden Ansprüche,
**dadurch gekennzeichnet,**
**dass** der erste und der zweite Farbbereich (2, 3), insbesondere durch Kennzeichnung unterschiedlicher Schraublöcher (5) oder unterschiedliche Schraubenlöchergruppen, eine Fixationsreihenfolge oder unterschiedliche Verschraubungsvarianten kennzeichnend ausgebildet sind.

6. Implantat nach einem der vorhergehenden Ansprüche,
**dadurch gekennzeichnet,**
**dass** der erste Farbbereich (2) eine erste Skalierung, insbesondere eine erste Bohrtiefe und/oder der zweite Farbbereich (3) eine zweite Skalierung, insbesondere eine zweite Bohrtiefe, kennzeichnend ausgebildet sind/ist.

7. Implantat nach einem der vorhergehenden Ansprüche,
**dadurch gekennzeichnet,**
**dass** der erste Farbbereich (2) einen biegsamen Implantatbereich und/oder der zweite Farbbereich (3) einen starren Implantatbereich kennzeichnend ausgebildet sind/ist.

8. Implantat nach einem der vorhergehenden Ansprüche,
**dadurch gekennzeichnet,**
**dass** die Flächenerstreckung des ersten und des zweiten Farbbereichs (2, 3) jeweils mindestens 1 cm², vorzugsweise mindestens 2 cm², betragen.

9. Implantat nach einem der vorhergehenden Ansprüche,
**dadurch gekennzeichnet,**
**dass** die unterschiedliche Farbgebung durch unterschiedliche zusammengesetzte Elektrolysebäder und/oder unterschiedliche, vorzugsweise um mindestens 1V, bevorzugt um mindestens 2V, noch weiter bevorzugt um mindestens 5V unterschiedliche, elektrische Elektrolysespannungen und/oder eine unterschiedliche Elektrolysedauer verursacht sind.

10. Sortiment aus mindestens zwei unterschiedlichen Implantaten nach einem der vorhergehenden Ansprüche,
**dadurch gekennzeichnet,**
**dass** die Farbkombinationen der Implantate (1) unterschiedlich sind und/oder oder die Größenverhältnisse der Farbbereiche und/oder die Konturierung der miteinander kombinierten Farbbereiche und/oder die Anzahl der unterschiedlichen miteinander kombinierten Farben.

11. Verfahren zum Herstellen eines medizinischen Implantates (1) nach einem der Ansprüche 1 bis 9 mit den folgenden Schritten:
● Bereitstellen eines Implantatkörperrohlings,
● Elektrolytische Bearbeitung eines ersten Oberflächenbereichs bei einer ersten elektrischen Spannung und/oder über eine erste Zeitdauer und/oder in einem ersten Elektrolysebad zur Erzeugung der ersten Oxidschicht (11) und damit des ersten Farbbereichs (2) der ersten Farbe,
● Elektrolytische Bearbeitung eines zweiten Oberflächenbereichs zur Erzeugung der zweiten Oxidschicht (12) und damit des zweiten Farbbereichs (3) der zweiten Farbe.

12. Verfahren nach Anspruch 11,
**dadurch gekennzeichnet,**
**dass** sich der erste und der zweite Oberflächenbereich nicht, teilweise oder vollständig überlappen.

13. Verfahren nach einem der Ansprüche 11 oder 12,
**dadurch gekennzeichnet,**
**dass** die elektrolytische Bearbeitung des zweiten Oberflächenbereichs bei keiner, teilweiser oder vollständiger Überlappung mit dem ersten Oberflächenbereich bei einer von der ersten elektrischen Spannung unterschiedlichen zweiten elektrischen Spannung und/oder in einem von dem ersten Elektrolysebad unterschiedlichen zweiten Elektrolysebad und/oder über eine von der ersten Zeitdauer unterschiedlichen zweiten Zeitdauer, durchgeführt wird, oder dass die elektrolytische Bearbeitung des zweiten Oberflächenbereichs bei teilweiser oder vollständiger Überlappung mit dem ersten Oberflächenbereich bei der ersten elektrischen Spannung und/oder über die erste Zeitdauer und/oder im ersten Elektrolysebad durchgeführt wird.

14. Verfahren nach einem der Ansprüche 11 bis 13,
**dadurch gekennzeichnet,**
**dass** der Implantatkörperrohling während der Erzeugung des ersten oder zweiten Farbbereichs (2) nur abschnittsweise in das erste Elektrolysebad getaucht ist und/oder dass ein Implantatkörperrohlingabschnitt, der bei dem ersten oder zweiten Elektrolyseschritt zur Herstellung des ersten oder zweiten Farbbereichs (2) nicht eingefärbt werden soll, beispielsweise mit einer Schutzschicht, geschützt, insbesondere mittels eines die Schutzschicht bildenden Schutzbandes abgeklebt oder mit einem entfernbaren Schutzlack überdeckt, insbesondere übersprüht oder überdeckt, wird.

15. Verfahren nach einem der Ansprüche 12 bis 14,
**dadurch gekennzeichnet,**
**dass** der Implantatkörperrohling zur elektrolytischen Bearbeitung des ersten Oberflächenbereichs nur mit diesem mit dem ersten Elektrolysebad in Kontakt gebracht wird, vorzugsweise in dem die gesamte Implantatkörperrohlingoberfläche dem ersten Elektrolysebad ausgesetzt wird und dass in einem weiteren Verfahrensschritt der zweite Oberflächenabschnitt mit dem ersten oder dem zweiten Elektrolysebad in Kontakt gebracht wird, wobei der zweite Oberflächenbereich zumindest teilweise, vorzugsweise vollständig auf den ersten Oberflächenbereich angeordnet ist und bevorzugt kleiner ist als der erste Oberflächenbereich.
